# EUROPÄISCHE PATENTANMELDUNG

(11) **EP 1 944 060 A1**
(43) Veröffentlichungstag der Anmeldung: **16.07.2008**
(21) Anmeldenummer: 07023194.9
(22) Anmeldetag: 30.11.2007
(51) Int. Cl.: A61N 1/372

(54) **Patientengerät für die drahtlose Datenkommunikation mit einem Implantat**

(30) Priorität: 11.01.2007 DE 102007001705
(71) Anmelder: BIOTRONIK CRM Patent AG, 6341 Baar (CH)
(72) Erfinder: Lang, Martin, Dr., 91085 Weisendorf (DE)
(74) Vertreter: Lindner-Vogt, Karin L.

(57) **Zusammenfassung**

Patientengerät (10) mit einer ersten Schnittstelle (20) für die drahtlose Übertragung von Daten von und zu einem Implantat sowie mit einer zweiten Schnittstelle (22) für die Datenfernkommunikation mit einem zentralen Service-Center und mit einer dritten, von der ersten und zweiten Schnittstelle verschiedene Schnittstelle (24), wobei die dritte Schnittstelle (24) für den Anschluss eines Programmiergerätes an das Patientengerät (10) ausgebildet und das Patientengerät (10) ausgebildet ist, über die dritte Schnittstelle (24) seitens eines Programmiergerätes empfangene Programmierdaten an die erste Schnittstelle (20) weiterzuleiten und drahtlos zu einem Implantat zu übertragen.

## Beschreibung

Die Erfindung betrifft ein Patientengerät, welches über eine drahtlose, bidirektionale Schnittstelle für die bidirektionale Übertragung von Daten zwischen dem Patientengerät und einem Implantat wie beispielsweise einem Herzschrittmacher, implantierbaren Cardioverter/Defibrillator (ICD) oder dergleichen aufweist. Außerdem weist das Patientengerät eine zweite Schnittstelle für die Datenfernkommunikation mit einem zentralen Service-Center auf.

Derartige Patientengeräte sind in verschiedener Form grundsätzlich bekannt und erlauben es, einen Herzschrittmacher- oder Defibrillator-Patienten auch außerhalb einer Arztpraxis oder eines Krankenhauses hinsichtlich der Funktion des Implantats oder der vom Implantat erfassten physiologischen Daten zu überwachen. Moderne Implantate der genannten Art, implantierbare Herzschrittmacher oder implantierbare Cardioverter/Defibrillatoren, sind in der Lage, eine Vielzahl physiologischer Daten zu erfassen, die Aufschluss über die Entwicklung der jeweiligen (Herz-) Krankheit des Patienten geben. Auf deren Grundlage kann dass das jeweilige Implantat selbstständig oder nach korrigierenden beziehungsweise programmierendem Eingriff des Arztes eine jeweils bestmögliche Therapie für den Patienten liefern. Zu den seitens des Implantats erfassten Daten gehören beispielsweise intrakardiale Elektrokardiogramme, die mit Hilfe eines entsprechenden Implantats und dem dazugehörigen Patientengerät zu einem zentralen Service-Center fernübertragen werden können und dort von einem Arzt ausgewertet oder von dort aus einem Arzt zur Auswertung zugänglich gemacht werden können. Andere Daten, die vom Implantat zum Service-Center übertragen werden können, sind beispielsweise Betriebsdaten des Implantates wie dessen Batteriezustand oder dergleichen. Zur Übertragung dieser Daten weist das Patientengerät zum einen eine erste, implantatseitige Schnittstelle auf, die mit einem Sender-/Empfänger (Transceiver) zur Kommunikation mit einer entsprechenden Schnittstelle des Implantats ausgebildet ist. Außerdem weist das Patientengerät eine zweite Schnittstelle für die Datenfernkommunikation mit dem Service-Center auf. Im einfachsten Fall ist diese zweite Schnittstelle ein Telefonmodem. Bekannte Alternativen sind beispielsweise alle denkbaren Varianten eines Internetanschlusses oder einer Datenverbindung über ein Mobilfunknetz.

Im Stand der Technik ist auch bereits in Erwägung gezogen worden, Programmierungen des Implantats über die beiden vorgenannten Schnittstellen des Patientengerätes vorzusehen. Auf diese Weise ergibt sich die Möglichkeit der Fernprogrammierung des Implantats über das Patientengerät vom Service-Center aus. Wohl, weil sich bei dieser denkbaren Variante zum einen technische Probleme ergeben, wie die Frage der Datensicherheit und -integrität, aber auch weil bei der Fernprogrammierung eines Implantats seitens des Service-Centers beim Patienten selbst in der Regel kein betreuender Arzt zur Stelle ist, der im Zweifelsfalle sofort helfend eingreifen kann, hat sich die genannte Fernprogrammierung des Implantats über ein Service-Center bisher nicht durchsetzen können.

Ein heute übliches Szenario sieht daher wie folgt aus: Im zentralen Service-Center wird beispielsweise ein intrakardiales EKG, das einen kritischen Zustand des Patienten repräsentiert, oder es werden Betriebsdaten des Implantates, die eine dringende Handlung erforderlich machen, empfangen. In diesem Falle wird der den jeweiligen Patienten betreuende Arzt durch das Service-Center umgehend informiert und kann den betroffenen Patienten zu sich bestellen oder persönlich besuchen. Alsdann kann der Arzt weitere Diagnosen in unmittelbarer Nähe zum Patienten durchführen und möglicherweise erforderliche weitere Abfragen am Implantat oder Umprogrammierungen des Implantats mit Hilfe eines üblichen Programmiergerätes vornehmen. Die Datenkommunikation zwischen Programmiergerät und Implantat erfolgt ebenfalls drahtlos, jedoch über eine andere, wesentlich kurzreichweitigere, induktive Datenverbindung zwischen Implantat und einem Programmierkopf des Programmiergerätes. Dieser Programmierkopf des Programmiergerätes muss für die Datenkommunikation mit dem Implantat in die unmittelbare Nähe des Implantats gebracht und in der Regel direkt auf die Haut des Patienten aufgelegt werden. Der Vorteil einer derartig kurzreichweitigen Datenkommunikation besteht darin, dass die Gefahr von Interferenzen und Störungen der Datenübertragungsstrecke recht gering ist. Da die Nähe eines Arztes in den meisten Fällen ohnehin erwünscht ist, erscheint das Erfordernis der Nähe eines Arztes ebenfalls nicht als Nachteil.

Dennoch besteht das Bedürfnis, dass dem Stand der Technik entsprechende Szenario zu verbessern, ohne dafür die Nachteile der vorgeschlagenen Alternativen in Kauf nehmen zu müssen.

Erfindungsgemäß wird hierzu ein Patientengerät vorgeschlagen, welches neben den beiden genannten Schnittstellen eine dritte Schnittstelle aufweist, die für den Anschluss eines - vorzugsweise herkömmlichen - Programmiergerätes hergerichtet ist. Das Patientengerät wiederum ist hergerichtet, über die dritte Schnittstelle seitens des Programmiergerätes empfangene Programmierdaten auf die erste (drahtlose) Schnittstelle für die Kommunikation mit dem Implantat weiterzuleiten und drahtlos zu einem Implantat zu übertragen.

Auf diese Weise kann das Patientengerät den üblichen Programmierkopf eines Programmiergerätes ersetzen und im Implantat kann die zweite Datenkommunikationsschnittstelle für drahtlose Datenkommunikation mit dem Programmierkopf eingespart werden. Gleichzeitig steht dem Arzt immer die volle Funktionalität des Programmiergerätes zur Verfügung. Da das Patientengerät so gestaltet ist, dass es für den Datenaustausch zwischen Programmiergerät und Implantat quasi transparent ist, also den Datenaustausch in beide Richtungen ungehindert und ohne Veränderung der Daten zulässt, ist die Kompatibilität zwischen Programmiergerät und Implantat vollends gewahrt und es kommt zu keinerlei funktioneller Einschränkung im Umgang mit dem Programmiergerät. Der Vorteil einer relativ kurzreichweitigen Telemetrie zwischen Patientengerät und Implantat und den damit verbundenen Vorteilen hinsichtlich der Störungssicherheit bleibt erhalten.

Als bevorzugte Schnittstelle zwischen Patientengerät und Programmiergerät bieten sich alternativ sowohl drahtlose Schnittstellen, wie beispielsweise Bluetooth, oder drahtgebundene Schnittstellen, wie USB, oder auch eine patientengerätseitige Schnittstelle an, die der üblichen Schnittstelle zwischen Programmiergerät und Programmierkopf entspricht.

Im Hinblick auf die implantatseitige Schnittstelle des Patientengerätes ist es vorteilhaft, wenn diese nicht in das Patientengerät selbst integriert ist, sondern in ein relativ kleineres Hand- oder Mobilteil, welches vorzugsweise nicht größer ist, als eine Handfläche und welches es leicht macht, mit dem Mobilteil eine geringe Distanz zum Implantat herzustellen um auf diese Weise die Übertragungssicherheit noch einmal zu erhöhen. Auch das Patientengerät selbst ist üblicherweise tragbar und in diesem Sinne mobil. Es ist jedoch in der Regel voluminöser als das vorgeschlagene Mobilteil. Das Mobilteil ist vorzugsweise über eine Drahtverbindung mit dem Basisteil des Patientengerätes verbunden.

Hinsichtlich des inneren Aufbaus des Patientengerätes beziehungsweise des Basisteils des Patientengerätes bieten sich unter anderem zwei Alternativen an. Zum einen kann die dritte Schnittstelle für den Anschluss des Programmiergerätes direkt und unmittelbar mit der ersten, drahtlosen Schnittstelle für die Datenkommunikation mit dem Implantat verbunden sein. Auf diese Weise ist die gewünschte Transparenz des Programmiergerätes auch hardwaremäßig am konsequentesten verwirklicht. "Direkt" heißt in diesem Zusammenhang, dass zwischen der ersten und dritten Schnittstelle keine potentiell datenverändernden Bestandteile des Patientengerätes angeordnet sind. "Unmittelbar" heißt in diesem Zusammenhang, dass die Daten zwischen der ersten und dritten Schnittstelle keine Verzögerung z.B. durch Zwischenspeichern erfahren.

Zum anderen kann auch die dritte Schnittstelle für den Anschluss des Programmiergerätes mit einer zentralen Steuereinheit des Patientengerätes verbunden sein, die ihrerseits auch mit der ersten und zweiten Schnittstelle des Patientengerätes verbunden ist. In diesem Falle ist die zentrale Steuereinheit als potentiell datenverändernder und potentiell datenverzögernder Bestandteil des Patientengerätes so gestaltet, dass sie Programmierbefehle des Programmiergerätes sowie Daten vom Implantat zum Programmiergerät ohne unnötige Veränderung oder Verzögerung weiterleitet.

Um die übliche Fernüberwachung durch ein zentrales Service-Center mit Hilfe des Patientengerätes zu erlauben, weist dies vorzugsweise einen Datenspeicher auf, der mit der zentralen Steuereinheit des Patientengerätes verbunden ist und es erlaubt, Daten seitens des Implantats unabhängig davon aufzunehmen, ob die Service-Center-seitige Datenfernübertragung gerade zur Verfügung steht. Daten des Implantats können dann im Speicher des Patientengerätes zwischengespeichert werden und übertragen werden, wenn eine Verbindung für die Datenfernkommunikation mit dem zentralen Service-Center zur Verfügung steht. Letzteres kann beispielsweise nur einmal am Tag zu bestimmten Zeiten der Fall sein, während eine Datenkommunikation zwischen Implantat und Patientengerät auch mehrfach am Tag stattfinden soll.

Ein Patientengerät der erfindungsgemäßen Art erlaubt somit gleichermaßen die drei folgenden Betriebsszenarien:

Zum einen ist der übliche Fernüberwachungsbetrieb möglich, bei dem die Datenübertragung zwischen Implantat und Patientengerät einerseits sowie die Datenfernübertragung zwischen Patientengerät und zentralen Service-Center andererseits zu unterschiedlicher Zeit unter Zwischenspeicherung der jeweils übertragenen Daten in einem Speicher des Patientengerätes erfolgt.

Ein Patientengerät der erfindungsgemäßen Art erlaubt es darüber hinaus, eine Programmierung des Implantats auch vom entfernten, zentralen Service-Center durchzuführen, wie dies im Stand der Technik bereits vorgeschlagen wurde.

Schließlich vereinfacht das erfindungsgemäße Patientengerät die Programmierung des Implantats sowie die Kommunikation mit dem Implantat mit Hilfe eines Programmiergerätes, für welches das Patientengerät praktisch die Funktion eines Programmierkopfes übernimmt.

Weitere Vorteile und Merkmale des erfindungsgemäßen Programmiergerätes ergeben sich aus der nachfolgenden Beschreibung von Ausführungsbeispielen.

In diesem Sinne soll nun die Erfindung anhand von Ausführungsbeispielen mit Bezug auf die Figuren näher erläutert werden. Von den Figuren zeigen:

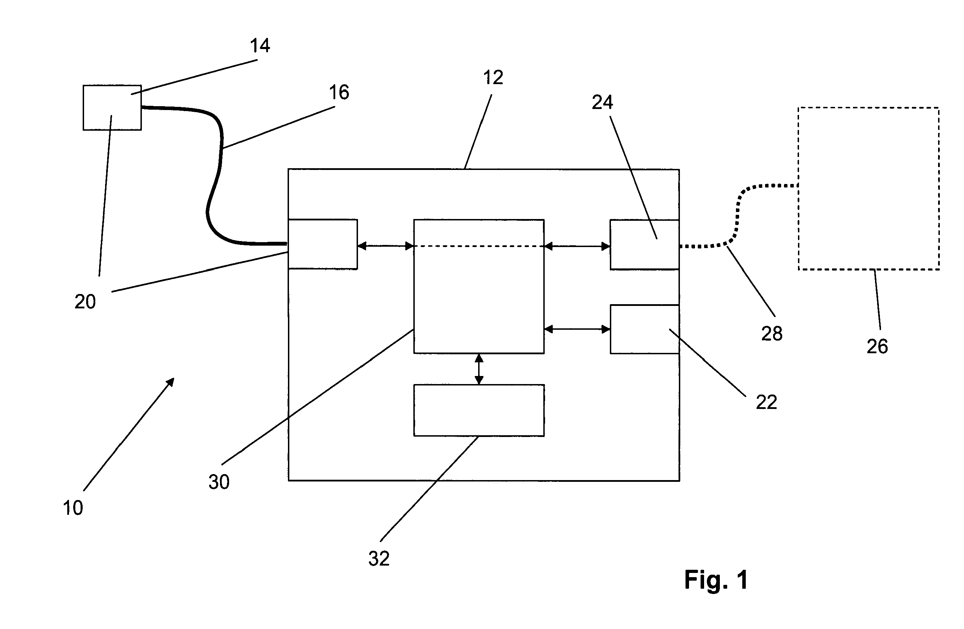

Fig. 1: eine erste Variante eines erfindungsgemäßen Programmiergerätes; und
Fig.: 2 eine zweite Variante eines erfindungsgemäßen Programmiergerätes.

In Fig. 1 ist ein erfindungsgemäßes Patientengerät 10 dargestellt, welches ein mobiles Basisteil 12 und ein Mobilteil 14 aufweist, welches über ein Kabel 16 an dem mobilen Basisteil angeschlossen ist.

Das Mobilteil 14 ist etwa handflächengroß und damit deutlich kleiner, als das Basisteil 12. Das Basisteil 12 kann beispielsweise etwa die Größe eines Tischtelefons haben.

Das Patientengerät 10 weist drei Schnittstellen auf, nämlich eine erste Schnittstelle 20 für die drahtlose Übertragung von Daten von und zu einem Implantat. Die erste Schnittstelle 20 ist bei dem Patientengerät 10 doppelt vorhanden, nämlich zum einen im Basisteil 12 und zum anderem im Mobilteil 14. Das Mobilteil 14 repliziert somit die erste Stelle 20 des Patientengerätes 10.

Des Weiteren ist eine zweite Schnittstelle 22 für die Datenfernkommunikation mit einem zentralen Service-Center vorhanden. Diese zweite Schnittstelle kann beispielsweise ein Telefonmodem sein oder jede andere Art von Schnittstelle, die eine Datenfernübertragung beispielsweise über das Internet zulässt.

Schließlich weist das Patientengerät 10 eine dritte Schnittstelle 24 auf, die zum Anschluss eines Programmiergerätes 26 dient, welches in Fig. 1 gestrichelt dargestellt ist. Das Programmiergerät 26 ist in dem in Fig. 1 abgebildeten Beispiel über ein Kabel 28 mit dem Patientengerät 10 verbunden. Alternativ könnte die dritte Schnittstelle 24 auch als Bluetooth-Transceiver ausgebildet sein, um mit dem Programmiergerät 26 drahtlos kommunizieren zu können.

Das Patientengerät 10 weist im Basisteil 12 eine zentrale Steuereinheit 30 auf, die mit allen drei Schnittstellen 20, 22 und 24 verbunden ist und darüber hinaus mit einem Speicher 32 verbunden ist.

Das Patientengerät 10 kann somit in herkömmlicher Weise zur Fernüberwachung eines Implantats verwendet werden. Dazu sendet das Implantat regelmäßig Daten an die erste Schnittstelle 20. Die Daten werden dann im Speicher 32 des Patientengerätes 10 gespeichert und können zu einem späteren Zeitpunkt vom zentralen Service-Center aus abgerufen werden. Dazu stellt das zentrale Service-Center eine Datenfernverbindung zum Patientengerät 10 über die zweite Schnittstelle 20 her.

Außerdem ist wie bei herkömmlichen Patientengeräten die Möglichkeit gegeben, dass ein Implantat vom zentralen Service-Center aus fernprogrammiert wird. In diesem Falle erfolgt die Datenübertragung vom zentralen Service-Center zum Implantat mit dem Patientengerät 10 als Relaisstation und es sind beide Schnittstellen, nämlich die erste Schnittstelle und die zweite Schnittstelle 22, gleichzeitig aktiv. Auf gleiche Weise kann auch eine unmittelbare Datenabfrage von Implantatfragen durch das zentrale Service-Center erfolgen.

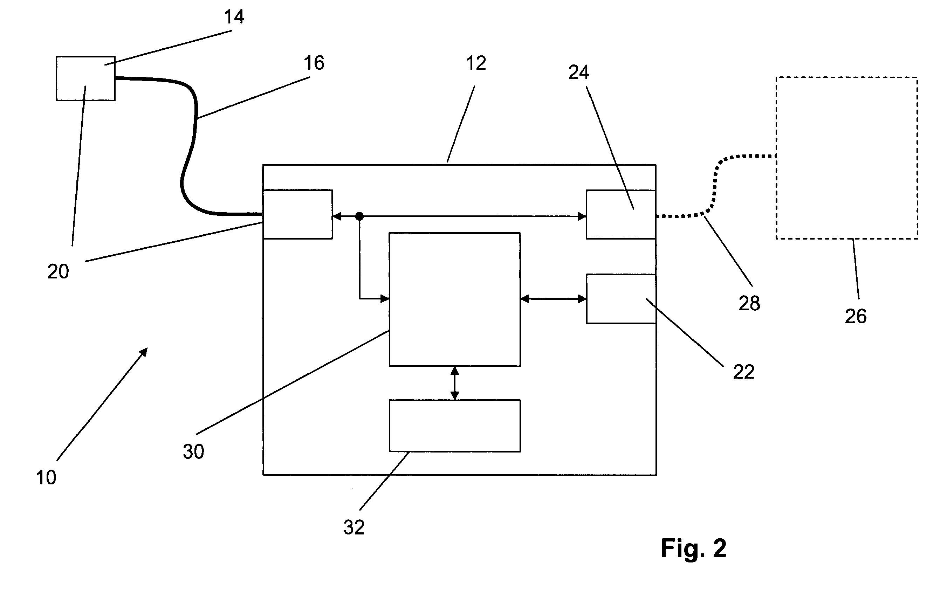

Darüber hinaus eröffnet das Patientengerät 10 die neue Möglichkeit, an das Patientengerät 10 ein Programmiergerät wie das Programmiergerät 26 anzuschließen und mit Hilfe des Programmiergerätes 26 eine Programmierung eines Implantats über die erste Schnittstelle 20 vorzunehmen. Dazu wird das Programmiergerät 26 an die dritte Schnittstelle 24 des Patientengerätes 10 angeschlossen. Das Patientengerät 10 ist so ausgebildet, dass das Programmiergerät 26 in diesem Falle einen transparenten oder auch einen tatsächlich direkten Datenzugriff auf die erste Schnittstelle 20 hat und somit über diese Daten von und zum Implantat übertragen kann. Um für diesen Zweck eine möglichst hohe Störsicherheit zu erlangen, ist bei dem Patientengerät 10 anders als bei bekannten Patientengeräten das Mobilteil 14 vorgesehen, welches einen Transceiver für die erste Schnittstelle 20 beherbergt und, falls notwendig, in unmittelbare Nähe des Implantats gebracht werden kann, so dass de facto nur eine kurze Funkübertragungsstrecke zwischen Implantat und Mobilteil 14 störungsfrei betrieben werden muss. Ist das Patientengerät 10 hinreichend kompakt und beweglich ausgebildet, kann das Mobilteil 14 auch entfallen. Die Ausführungsbeispiele in Fig. 1 und Fig. 2 unterscheiden sich nur dadurch, wie die dritte Schnittstelle 24 für den Anschluss des Programmiergerätes 26 mit der ersten Schnittstelle 20 für die drahtlose Datenübertragung von und zum Implantat verbunden ist.

Bei der Ausführungsvariante gemäß Fig. 1 ist die Datenverbindung zwischen der dritten und der ersten Schnittstelle transparent über die zentrale Steuereinheit 30 geführt.

Bei der Ausführungsvariante gemäß Fig. 2 ist die Verbindung zwischen der dritten Schnittstelle 24 und der ersten Schnittstelle 20 direkt verwirklicht.

## Patentansprüche

1. Patientengerät (10) mit
einer ersten Schnittstelle (20) für die drahtlose Übertragung von Daten von und zu einem Implantat sowie mit
einer zweiten Schnittstelle (22) für die Datenfernkommunikation mit einem zentralen Service-Center und mit
einer dritten, von der ersten und zweiten Schnittstelle verschiedene Schnittstelle (24),
**dadurch gekennzeichnet, dass** die dritte Schnittstelle (24) für den Anschluss eines Programmiergerätes an das Patientengerät (10) ausgebildet und dass das Patientengerät (10) ausgebildet ist, über die dritte Schnittstelle (24) seitens eines Programmiergerätes empfangene Programmierdaten an die erste Schnittstelle (20) weiterzuleiten und drahtlos zu einem Implantat zu übertragen.

2. Patientengerät nach Anspruch 1, **dadurch gekennzeichnet, dass** die dritte Schnittstelle (24) als drahtgebundene Schnittstelle für den Anschluss eines Programmiergerätes mittels eines Kabels ausgebildet ist.

3. Patientengerät gemäß Anspruch 1, **dadurch gekennzeichnet, dass** die dritte Schnittstelle (24) als drahtlose Schnittstelle für die drahtlose Übertragung von und zu einem Programmiergerät ausgebildet ist.

4. Patientengerät nach Anspruch 3, **dadurch gekennzeichnet, dass** die dritte Schnittstelle 24 über einen Bluetooth-Transceiver verfügt.

5. Patientengerät nach einem der Ansprüche1 bis 4, **gekennzeichnet durch** ein bewegliches Basisteil (12) und ein an das Basisteil (12) über eine drahtlose oder eine drahtgebundene Datenverbindung angebundenes Mobilteil (14), welches kleiner ist als das Basisteil (12) und die erste Schnittstelle (20) beherbergt oder eine der ersten Schnittstelle (20) entsprechende Schnittstelle aufweist.

6. Patientengerät nach Anspruch 5, **dadurch gekennzeichnet, dass** das Mobilteil (14) über eine Kabelverbindung (16) an das Basisteil (12) angeschlossen ist.

7. Patientengerät nach einem der Ansprüche 1 bis 6, **dadurch gekennzeichnet, dass** die dritte Schnittstelle (24) in einer Weise direkt mit der ersten Schnittstelle (20) verbunden ist, dass zwischen der dritten Schnittstelle (24) und der ersten Schnittstelle (20) keine Veränderung übertragener Daten erfolgt.

8. Patientengerät nach Anspruch 7, **dadurch gekennzeichnet, dass** die dritte Schnittstelle (24) mit der ersten Schnittstelle (20) derart unmittelbar verbunden ist, dass Daten zwischen der dritten Schnittstelle (24) und der ersten Schnittstelle (20) sowie umgekehrt keine Verzögerung erfahren.
